# EUROPÄISCHE PATENTSCHRIFT

(11) **EP 3 092 053 B1**
(45) Veröffentlichungstag und Bekanntmachung des Hinweises auf die Patenterteilung: **25.10.2017**
(21) Anmeldenummer: 15700038.1
(22) Anmeldetag: 05.01.2015
(51) Int. Cl.: B01D 53/14, B01D 3/14, C07C 41/42, C07C 41/40

(54) **VERFAHREN ZUR TRENNTECHNISCHEN BEARBEITUNG EINES PRODUKTSTROMS EINES DIMETHYLETHERREAKTORS**
METHOD FOR THE SEPARATION OF A STREAM OF PRODUCTS FROM A DIMETHYL ETHER REACTOR
PROCÉDÉ DE SÉPARATION D'UN FLUX DE PRODUIT D'UN RÉACTEUR À DIMÉTHYLÉTHER

(30) Priorität: 07.01.2014 EP 14000041
(43) Veröffentlichungstag der Anmeldung: 16.11.2016
(73) Patentinhaber: Linde Aktiengesellschaft, 80331 München (DE)
(72) Erfinder: FRITZ, Helmut, 81375 München (DE); PESCHEL, Andreas, 82515 Wolfratshausen (DE)
(86) Internationale Anmeldenummer: PCT/EP2015/050046
(87) Internationale Veröffentlichungsnummer: WO 2015/104238

(56) Entgegenhaltungen:
- EP-A2- 0 343 454
- DE-A1- 19 943 219
- US-A- 5 189 203
- US-A1- 2013 327 086

## Beschreibung

Die Erfindung betrifft ein Verfahren zur trenntechnischen Bearbeitung eines zumindest Dimethylether, Methanol, Wasser, Kohlendioxid, Kohlenmonoxid und Wasserstoff enthaltenden Produktstroms eines Reaktors, der zur Synthese von Dimethylether aus Synthesegas verwendet wird,sowie eine Anlage zur Erzeugung von Dimethylether.

### Stand der Technik

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether kann entweder direkt aus Methanol oder indirekt aus Erd- oder Biogas erzeugt werden. Im letzteren Fall wird das Erd- oder Biogas zunächst zu Synthesegas reformiert. Synthesegas kann auch mittels anderer Verfahren, beispielsweise durch Pyrolyse von Abfällen oder Biomasse, gewonnen werden. Das Synthesegas wird anschließend entweder in einer zweistufigen Reaktion zu Methanol und anschließend zu Dimethylether oder in einer einstufigen Reaktion direkt zu Dimethylether konvertiert.

Die Synthese von Dimethylether aus Synthesegas ist thermodynamisch und wirtschaftlich vorteilhaft gegenüber einer Synthese aus Methanol.

Die vorliegende Erfindung betrifft insbesondere die einstufige Synthese von Dimethylether, wobei unter einer "einstufigen" Synthese eine Synthese verstanden wird, bei der sämtliche Reaktionen in einunddemselben Reaktor ablaufen. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 1 00 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor enthaltener Produktstrom wird kopfseitig entnommen, gekühlt und einer Trennung zugeführt.

Der Produktstrom enthält neben Dimethylether nicht umgesetzte Komponenten des Synthesegases sowie weitere Reaktionsprodukte. Typischerweise weist der Produktstrom neben Dimethylether zumindest Methanol, Wasser, Kohlendioxid, Kohlenmonoxid und Wasserstoff sowie in geringerer Menge Methan, Ethan, organische Säuren und höhere Alkohole auf.

Der Produktstrom wird bei dem zuvor erwähnten Druck zwischen 20 und 80 bar erhalten. Zur Gewinnung von Dimethylether aus dem Produktstrom muss dieser auf Temperaturen deutlich unter 0°C abgekühlt werden. Um hierbei beispielsweise das Ausfrieren von Wasser zu vermeiden und/oder um Dimethylether spezifikationsgerecht gewinnen zu können, kann es erforderlich sein, vor der Abkühlung des Produktstroms größere Mengen an Methanol und/oder Wasser abzutrennen.

Aufgrund der vergleichsweise hohen Löslichkeit von Dimethylether und Kohlendioxid in Methanol und Wasser sowie aufgrund des hohen Drucks lässt sich jedoch trotz der hohen Siedepunktsunterschiede der genannten Komponenten mit einer einstufigen partiellen Kondensation keine befriedigende Trennung erreichen.

Aus der US 2013/327086 A1 ist ein Verfahren zur vereinfachten Abtrennung eines Reaktionsprodukts aus einem Reaktionsgasgemisch, bestehend aus dem Reaktionsprodukt, mindestens einem Leichtsieder und/oder nicht kondensierbaren Reaktionsgas und mindestens einem Hochsieder, bekannt. Die EP 0 343 454 A2 betrifft ein in eine Methanolsynthese integriertes Verfahren zur Herstellung von Dimethylether durch katalytische Dehydratisierung von Methanol und die Reinigung des Dehyhdratisierungsprodukts durch Zuführung desselben in eine Destillationskolonne zur Gewinnung von reinem Dimethylether. Zur Erhöhung der Ausbeute an Propan, Butan und anderer schwereren Komponenten aus einem Erdgasstrom wird in der US 5 685 170 A der Einsatz einer Absorptionskolonne vorgeschlagen. Ein Verfahren zur Herstellung von Dimethylether ist beispielsweise auch aus der DE 199 43 219 A1 bekannt, die als nächstliegender Stand der Technik angesehen wird und ein Verfahren zur Gewinnung von Dimethylether aus dem bei der Synthese von Dimethylether aus Synthesegas anfallenden Produktstrom wobei der Produktstrom zumindest teilweise gasförmig in eine mit einem flüssigen Rücklauf betriebene Absorptionskolonne eingespeist wird, der Absorptionskolonne ein gasförmiger Kopfstrom und ein flüssiger Sumpfstrom entnommen werden, zumindest ein Teil des Sumpfstroms in eine erste Destillationssäule in einen Dimethylether enthaltenden Strom und einen überwiegend Methanol und/oder Wasser enthaltenden Strom getrennt wird, und der Kopfstrom der Absorptionskolonne zumindest teilweise in einer weitere Destillationssäule aufgetrennt wird. Es besteht der Bedarf nach verbesserten Möglichkeiten zur Reduzierung des Methanol- und/oder Wassergehalts eines entsprechenden Produktstroms, insbesondere bei dem zuvor erwähnten Druck.

### Offenbarung der Erfindung

Vor diesem Hintergrund schlägt die vorliegende Erfindung ein Verfahren zur trenntechnischen Bearbeitung eines zumindest Dimethylether, Methanol, Wasser, Kohlendioxid, Kohlenmonoxid und Wasserstoff enthaltenden Produktstroms eines Reaktors, der zur Synthese von Dimethylether aus Synthesegas verwendet wird, sowie eine Anlage zur Herstellung von Dimethylether gemäß den Merkmalen der unabhängigen Patentansprüche vor. Bevorzugte Ausführungsformen sind Gegenstand der Unteransprüche sowie der nachfolgenden Beschreibung.
Vor der Erläuterung der Merkmale und Vorteile der vorliegenden Erfindung werden deren Grundlagen und die verwendeten Begriffe erläutert.
Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Ein flüssiger oder gasförmiger Strom ist von einem anderen flüssigen oder gasförmigen Strom (auch als Ausgangsstrom bezeichnet) "abgeleitet", wenn er zumindest einige in dem Ausgangsstrom enthaltene oder aus diesem erhaltene Komponenten aufweist. Ein in diesem Sinne abgeleiteter Strom kann aus dem Ausgangsstrom durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, auch als Sumpfprodukt bezeichnet, wird in einer Destillationssäule mittels eines Sumpfverdampfers erwärmt, so dass kontinuierlich ein Teil des Sumpfprodukts verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, auch als Kopfprodukt bezeichnet, eingespeist, verflüssigt und als flüssiger Rücklauf am Kopf der Destillationssäule aufgegeben wird.

Im Gegensatz zu einer Destillationssäule verfügt eine "Absorptionskolonne" nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen.

Zur Auslegung und spezifischen Ausgestaltung von Destillationssäulen und Absorptionskolonnen sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

Ist nachfolgend kurz von einer "Synthese" von Dimethylether die Rede, wird hierunter ein Verfahren verstanden, bei dem ein Synthesegas enthaltender Einsatz, also ein Gasgemisch, das in geeigneten Anteilen zumindest Kohlenmonoxid und Wasserstoff enthält, zu einem entsprechenden Dimethylether enthaltenden Produktstrom umgesetzt wird. Ein entsprechender Produktstrom enthält aufgrund der nicht vollständigen Reaktion und aufgrund des Auftretens von Nebenreaktionen bei der Synthese von Dimethylether, insbesondere in Abhängigkeit von den Charakteristika der verwendeten Katalysatoren und den jeweiligen Gehalten der Komponenten des Synthesegases, nicht ausschließlich Dimethylether sondern weitere Verbindungen. Diese sind zumindest Methanol, Wasser, Kohlendioxid, Kohlenmonoxid und Wasserstoff, jedoch typischerweise auch geringere Mengen Methan, Ethan, organische Säuren und höhere Alkohole. Diese genannten weiteren Verbindungen müssen, wie erwähnt, abgetrennt werden. Die Abtrennung erfolgt einerseits, um nachfolgende Trennschritte zu ermöglichen, und andererseits, um Dimethylether in der geforderten Reinheit, also "spezifikationsgerecht" zu gewinnen.

### Vorteile der Erfindung

Die vorliegende Erfindung geht von dem eingangs erläuterten Problem aus, das, wie erläutert, darin besteht, dass herkömmlicherweise zur Abtrennung von Dimethylether aus einem Produktstrom eines Reaktors zur Herstellung von Dimethylether dieser auf Temperaturen deutlich unter 0°C abgekühlt werden m uss. Um hier das Ausfrieren von Wasser zu vermeiden und/oder Dimethylether spezifikationsgerecht gewinnen zu können, müssen, wie erwähnt, größere Mengen an Methanol und Wasser abgetrennt werden. Dies erweist sich als aufwändig, weil sich Dimethylether und Kohlendioxid in Methanol und Wasser ausgesprochen gut lösen und sich bei den eingesetzten hohen Drücken trotz der Siedepunktsunterschiede mit einer einstufigen partiellen Kondensation keine befriedigende Trennung erreichen lässt. Ein Druckabbau auf geringere Drücke, beispielsweise Umgebungsdruck, der eine Trennung erleichtern würde, ist nachteilig, weil für die anschließende Trennung unter Tieftemperaturbedingungen wieder ein entsprechend hoher Druck zwischen 20 und 100 bar vorliegen muss. Es müsste also eine energieaufwendige Rückverdichtung erfolgen. Selbst eine mehrstufige Kondensation alleine führt aber nicht zu einer zufriedenstellenden Trennung.

Vor diesem Hintergrund schlägt die vorliegende Erfindung ein Verfahren zur trenntechnischen Bearbeitung eines entsprechenden Produktstroms, der zumindest Dimethylether, Methanol, Wasser, Kohlendioxid, Kohlenmonoxid und Wasserstoff enthält, vor. Wie erwähnt, stammt ein derartiger Produktstrom aus einem zur Synthese von Dimethylether aus Synthesegas verwendeten Reaktor, insbesondere einem Rohrreaktor, der mit Synthesegas beschickt wird und zur einstufigen Synthese von Dimethylether bzw. der zumindest teilweisen Umsetzung von Synthesegas zu Dimethylether eingerichtet ist.

Erfindungsgemäß ist vorgesehen, den Produktstrom zumindest teilweise gasförmig in eine mit einem flüssigen Rücklauf betriebene Absorptionskolonne einzuspeisen. Die Einspeisung erfolgt also, im Falle vollständig gasförmiger Einspeisung, oberhalb des Taupunkts des entsprechenden Produktstroms bzw. der Komponente mit dem höchsten Siedepunkt auf dem verwendeten Druckniveau. Es kann jedoch auch ein teilweise flüssiger Produktstrom in die Absorptionskolonne eingespeist werden, d.h. der Produktstrom kann auch "ankondensiert" sein.

Der Absorptionskolonne werden kopfseitig ein gasförmiger Kopfstrom und sumpfseitig ein flüssiger Sumpfstrom entnommen. Auch im Fall einer Einspeisung eines teilweise flüssigen Stroms gehen Gase wie Kohlendioxid, Kohlenmonoxid und Wasserstoff kaum in dem Sumpfstrom über, da die in der Absorptionskolonne verwendeten Temperaturen vergleichsweise hoch sind. Vorteilhafterweise ist die Eintrittstemperatur in die Absorptionskolonne wesentlich höher als die Temperatur in deren Kopf (letztere liegt typischerweise auf einem Temperaturniveau von 50 bis 150 °C). Dadurch lösen sich wesentlich weniger der genannten Gase im Sumpfstrom als dies beispielsweise bei einer einstufigen Kondensation der Fall wäre. Der Sumpfstrom ist daher in beiden Fällen arm an oder frei von den genannten Gasen, was die Trennung in den nachfolgend erläuterten Schritten deutlich verbessert.

Der der Absorptionskolonne entnommene Kopfstrom wird zumindest zum Teil zunächst auf ein erstes und anschließend weiter auf ein oder mehrere weitere Temperaturniveaus abgekühlt, wobei nach dem Abkühlen auf das erste Temperaturniveau ein erster Kondensatstrom gebildet wird und nach dem weiteren Abkühlen auf das oder die weiteren Temperaturniveaus ein oder mehrere weitere Kondensatströme gebildet werden. Die weitere Abkühlung kann also mehrstufig sein, wobei optional nach einer letzten Abkühlstufe auch eine weitere Absorptionskolonne zum Einsatz kommen kann, wie unten erläutert. Nach den sonstigen Abkühlstufen werden hingegen typischerweise normale Abscheidebehälter eingesetzt, um die Kondensatströme zu bilden. Ist hier davon die Rede, dass "der Kopfstrom" (oder ein Teil davon) mehrfach unter Bildung von Kondensaten abgekühlt wird, versteht sich, dass jede zusätzliche Abkühlstufe immer nur den nicht kondensierten Anteil (oder einen Teil davon) betrifft, also sich die Menge des abgekühlten Fluids kontinuierlich verringert.

Der Sumpfstrom aus der Absorptionskolonne wird zumindest zum Teil in eine erste Destillationssäule eingespeist, wobei der ersten Destillationssäule ein Dimethylether enthaltender Transferstrom und ein überwiegend Methanol und/oder Wasser enthaltender Strom entnommen werden. Der "Transferstrom" wird typischerweise aus Fluid gebildet, das der ersten Destillationssäule kopfseitig entnommen wird.

Der Transferstrom kann flüssig, teilweise flüssig oder gasförmig sein. Beispielsweise kann aus der ersten Destillationssäule zur Bereitstellung eines flüssigen Rücklaufs Fluid ausgeführt und teilweise in einem Kopfkondensator verflüssigt werden. Bei dem Transferstrom kann es sich um entsprechendes Fluid handeln, das nicht verflüssigt wird (oder einen Teil davon), aber auch um entsprechend verflüssigtes oder teilverflüssigtes Fluid. Der Transferstrom enthält neben Dimethylether damit typischerweise auch Kohlendioxid, ist aber vorzugsweise arm an oder frei von Wasser und/oder Methanol. Seine weitere Behandlung ist unten erläutert.

Der ebenfalls der Destillationssäule entnommene, überwiegend Methanol und/oder Wasser enthaltende Strom wird aufgrund der erfindungsgemäß vorgeschlagenen Maßnahmen nicht der Tieftemperaturtrennung zugeführt, in der Wasser und/oder Methanol, wie erwähnt, stören könnten.

Zumindest ein Teil des oder der weiteren Kondensatströme und des Transferstroms wird in eine weitere Destillationssäule eingespeist, der sumpfseitig ein flüssiger Strom entnommen wird, der überwiegend Dimethylether enthält und arm an oder frei von Kohlendioxid ist. Bei letzterem handelt es sich um das eigentliche Produkt des erfindungsgemäßen Verfahrens.

Der erste Kondensatstrom wird erfindungsgemäß teilweise zur Bildung des flüssigen Rücklaufs und teilweise anderweitig verwendet. Der nicht zur Bildung des flüssigen Rücklaufs verwendete Anteil des ersten Kondensatstroms kann, wenn er wasser- und ggf. methanolfrei ist, ebenfalls zumindest zum Teil in die weitere Destillationssäule eingespeist werden, in die zumindest ein Teil des oder der weiteren Kondensatströme und des Transferstroms eingespeist wird. Anderenfalls kann der nicht zur Bildung des flüssigen Rücklaufs verwendete Anteil des ersten Kondensatstroms auch in die erste Destillationssäule eingespeist werden, in die auch der flüssige Sumpfstrom aus der Absorptionskolonne eingespeist wird.

Mit anderen Worten wird die Absorptionskolonne im Rahmen der vorliegenden Erfindung mit einem flüssigen Rücklauf betrieben, der erfindungsgemäß aus einem Kondensatstrom gebildet wird, welcher aus dem kopfseitig der Absorptionskolonne entnommenen gasförmigen Kopfstrom nach Abkühlung auf ein erstes Temperaturniveau flüssig abgeschieden wird. Bei dem Kopfstrom handelt es sich um das Reinigungsprodukt der Absorptionskolonne, das zuvor im Gegenstrom zu dem flüssigen Rücklauf die Absorptionskolonne von unten nach oben durchlaufen hat.

Wird die erste Destillationssäule bei einem höheren Druck als die weitere Destillationssäule betrieben, wird der Transferstrom aus der ersten Destillationssäule vorteilhafterweise gasförmig oder flüssig bereitgestellt und kann direkt in die weitere Destillationssäule überführt werden. Wird die erste Destillationssäule hingegen bei einem niedrigeren Druck als die weitere Destillationssäule betrieben, wird vorteilhafterweise ein flüssiger Transferstrom verwendet, der mittels einer Pumpe druckerhöht werden kann. In diesem Fall kann ggf. ein gasförmiger, aus Fluid des Transferstroms bestehender Strom verbleiben, der beispielsweise zu einem Brenner geleitet werden kann. Typischerweise ist dessen Menge im Vergleich zum flüssigen Kondensatstrom, der in die weitere Destillationssäule überführt wird, gering. Die in der ersten und der weiteren Destillationssäule verwendeten Drücke werden vorteilhafterweise derart gewählt, dass in der ersten Destillationssäule möglichst Kühlwasser als Kühlmedium in deren Kopfkondensator verwendet werden kann und in der weiteren Destillationssäule eine effektive Trennung von Kohlendioxid und Dimethylether unter Verwendung eines Kühlmittels bei einer Temperatur von mehr als ca. -47 °C (höher als der Gefrierpunkt von Kohlendi oxid) möglich ist. Die Betriebsdrücke beider Destillationssäulen liegen unterhalb jener, bei der die Bildung des oder der weiteren Kondensate erfolgt.

Die oben erläuterte Trennaufgabe wird im Rahmen der vorliegenden Erfindung also unter anderem dadurch gelöst, dass der Produktstrom zunächst in eine Absorptionskolonne geleitet und mit zumindest einem Teil des anfallenden Kondensats einer ersten Kondensationsstufe als Rücklauf gewaschen wird. In die erste Kondensationsstufe wird der gasförmige Kopfstrom der Absorptionskolonne eingespeist. Die Temperatur der Kondensation in dieser ersten Kondensationsstufe, also das erste Temperaturniveau, bei der der Kondensatstrom abgeschieden wird, richtet sich dabei nach dem Taupunkt des Kopfstroms bzw. seiner Komponenten und den verfügbaren Kältemitteln, zum Beispiel Luft, Kühlwasser, C3-Kältemittel oder Dimethylether. In den Rücklauf der Absorptionskolonne und damit den Sumpfstrom gelangter Dimethylether geht erfindungsgemäß nicht verloren, sondern wird in dem Transferstrom aus der ersten Destillationssäule, in die der Sumpfstrom zuvor eingespeist wird, zumindest größtenteils in die weitere Destillationssäule überführt, die zur Bereitstellung des eigentlichen Dimethyletherprodukts dient. Gleichzeitig ermöglicht die vorliegende Erfindung, wie bereits erwähnt, eine Tieftemperaturtrennung ohne negative Auswirkungen von Wasser und/oder Methanol im Produktstrom, da diese Komponenten in der Absorptionskolonne ausgewaschen werden.

Das erfindungsgemäß vorgeschlagene Verfahren erweist sich als energetisch bedeutend günstiger als herkömmliche Verfahren, so dass durch die erfindungsgemäßen Maßnahmen eine vorteilhafte Trennung gegenüber Trennverfahren erzielt wird, wie sie aus dem Stand der Technik bekannt sind.

Vorteilhafterweise wird die Absorptionskolonne, insbesondere durch Einstellen der Temperatur- und Druckbedingungen und der Menge des verwendeten Rücklaufs, derart betrieben, dass der der Absorptionskolonne entnommene Kopfstrom arm an Methanol und/oder Wasser ist. Vorzugsweise ist dieser Kopfstrom weitgehend frei von Methanol und/oder Wasser.

Der der Absorptionskolonne entnommene Kopfstrom weist damit noch im Wesentlichen die anderen Komponenten des Produktstroms auf, nämlich zumindest Dimethylether, Kohlendioxid, Kohlenmonoxid und Wasserstoff. Ein Strom mit dieser Zusammensetzung erweist sich in den nachgeschalteten Trenneinheiten als unproblematisch, da er insbesondere kein möglicherweise ausfrierendes Wasser und die Trenneigenschaften negativ beeinflussendes Methanol mehr enthält.

Erfindungsgemäß ist, wie erwähnt, vorgesehen, einen auf dem ersten Temperaturniveau gasförmig verbleibenden Anteil des Kopfstroms aus der Absorptionskolonne sukzessive zumindest auf wenigstens ein weiteres, beispielsweise auf ein zweites und ein drittes Temperaturniveau, abzukühlen. Auf dem zweiten und dem dritten Temperaturniveau können dabei weitere Kondensatströme flüssig abgeschieden werden. Es werden also jeweils nach einer entsprechenden Abkühlung eine oder mehrere weitere Kondensationen durchgeführt. Eine stufenweise Abkühlung erweist sich als energetisch besonders günstig und ist beispielsweise zur Abtrennung von Ethan aus ethanhaltigen Gemischen bekannt.

Vorteilhafterweise werden bei der Abtrennung entsprechender weiterer Kondensatströme die verwendeten Temperaturniveaus derart gewählt, dass diese weiteren Kondensatströme arm an Kohlenmonoxid und Wasserstoff sind. Vorteilhafterweise enthalten diese Kondensatströme damit im Wesentlichen noch Kohlendioxid und Dimethylether, welche in einer nachgeschalteten Trennung voneinander getrennt werden können.

Hierzu ist erfindungsgemäß die weitere Destillationssäule vorgesehen, in welche ggf. der nicht als flüssige Rücklauf verwendete Anteil des Kondensatstroms (siehe oben), der aus dem gasförmigen Kopfstrom aus der Absorptionskolonne abgeschieden wird, jedenfalls aber zumindest einer der weiteren Kondensatströme sowie der Transferstrom zumindest teilweise eingespeist wird. Hierbei werden die in den weiteren Kondensationsstufen gewonnenen Kondensate und ggf. weitere eingespeiste Fluide in dieser weiteren Destillationssäule getrennt.

Dies erfolgt unter Bedingungen, die bewirken, dass der weiteren Destillationssäule sumpfseitig ein flüssiger Sumpfstrom entnehmbar ist, der reich an Dimethylether und arm an Kohlendioxid ist. Die Trennaufgabe der weiteren Destillationssäule kann also damit beschrieben werden, eine Trennung von Kohlendioxid und Dimethylether in einem entsprechenden Gemisch vorzunehmen. Der weiteren Destillationssäule wird kopfseitig ein gasförmiger Kopfstrom entnommen, welcher reich an Kohlendioxid und arm an Dimethylether ist.

Die vorliegende Erfindung eignet sich insbesondere für Verfahren, bei denen der Produktstrom aus dem zur Synthese von Dimethylether aus Synthesegas verwendeten Reaktor auf einem Druckniveau von 20 bis 100 bar, insbesondere auf einem Druckniveau von 30 bis 80 bar, in die Absorptionskolonne eingespeist wird. Die Abtrennung von Methanol und/oder Wasser kann hier unter Druck erfolgen, eine vorherige Entspannung, die dann eine erneute energieaufwendige Druckbeaufschlagung erfordern würde, ist nicht notwendig.

Die vorliegende Erfindung eignet sich für eine Trennung direkt im Anschluss an die Synthese und eine nachgeschaltete Abkühlung. Besonders vorteilhaft ist hierbei, wenn die Abkühlung nur durch einen Wärmetausch des Produktstroms mit einem in den oder die Reaktoren eingespeisten Synthesegasstrom erfolgt, so dass auf weitere aufwendige Kühleinrichtungen mit ggf. extern zugeführtem Kältemittel verzichtet werden kann. Dies ist möglich, weil der Taupunkt des Produktstroms im Rahmen der vorliegenden Erfindung nicht unterschritten werden muss. Der Produktstrom kann also trotz der Abkühlung in überhitztem Zustand bleiben, d.h. auf einem Temperaturniveau oberhalb des Taupunkts. Sein Temperaturniveau bei der Einspeisung in die Absorptionskolonne kann also beispielsweise bei 60 bis 150 °C, insbesondere bei 70 bis 120 °C, beispielsweise bei 80 bis 100°C oder, auf den Taupunkt bezogen, beispielsweise mindestens 10°C und maximal 30 bis 50 °C oberhalb des Taupunkts liegen. Wie erwähnt, ist auch eine Einspeisung in ankondensierter Form möglich.

Die anschließende sukzessive Abkühlung des Kopfstroms der Absorptionskolonne erfolgt auf immer niedrigere Temperaturen, vorteilhafterweise bis auf ein minimales Temperaturniveau, das zwischen der Schmelztemperatur von Kohlendioxid auf dem verwendeten Druckniveau und -15°C liegt, beispiels weise bei -50 bis -20°C und insbesondere bei ca. -35 °C, der Temperatur eines C 3-Kältemittels. Das Temperaturniveau kann dabei auch knapp, d.h. mindestens 0,5 bis 10 °C, insbesondere 1 bis 5 °C, oberhalb der Schmelztemper atur von Kohlendioxid auf dem verwendeten Druckniveau liegen. Das verwendete Temperaturniveau richtet sich auch nach der Zusammensetzung des Kopfstroms und der gewünschten Zusammensetzung der hierbei gewonnenen Kondensate. Auf diese Weise kann eine nahezu vollständige Abscheidung des Kohlendioxids und des Dimethylethers aus dem Kopfstrom der Absorptionskolonne erzielt werden.

Nach dem Abkühlen auf das minimale Temperaturniveau kann ein noch immer gasförmig verbliebener Anteil des Kopfstroms in eine weitere Absorptionskolonne eingespeist werden, die eine besonders effektive Abreicherung an Dimethylether ermöglicht. Hierzu kann die weitere Absorptionskolonne mit einem weiteren flüssigen Rücklauf betrieben werden, der aus einem verflüssigten, kohlendioxidreichen Kopfstrom der weiteren Destillationssäule gebildet wird.

Das erfindungsgemäße Verfahren kann mit Produktströmen unterschiedlichster Zusammensetzung zum Einsatz kommen. Entsprechende Produktströme enthalten beispielsweise 2 bis 50 Molprozent, insbesondere 5 bis 30 Molprozent, Dimethylether, 0,1 bis 20 Molprozent, insbesondere 0,7 bis 10 Molprozent, Methanol, 0,1 bis 20 Molprozent, insbesondere 0,8 bis 10 Molprozent, Wasser, 1 bis 50 Molprozent, insbesondere 3 bis 30 Molprozent, Kohlendioxid, 0,1 bis 25 Molprozent, insbesondere 1 bis 11 Molprozent Kohlenmonoxid und 5 bis 90 Molprozent, insbesondere 20 bis 80 Molprozent, Wasserstoff. Nach der Entfernung von Wasser und Methanol ist das Gasgemisch vorzugsweise arm an Wasser und Methanol.

Solche Produktströme werden beispielsweise durch Beschicken eines Reaktors mit einem Synthesegas erhalten, in dem das Verhältnis von Wasserstoff zu Kohlenmonoxid 0,8 bis 8 mol/mol, insbesondere 1 bis 6 mol/mol beträgt.

Die erfindungsgemäß vorgesehene Anlage zur Herstellung von Dimethylether profitiert von den zuvor erläuterten Vorteilen, auf die daher ausdrücklich verwiesen wird.

Die Erfindung wird unter Bezugnahme auf die Zeichnungen näher erläutert, die eine Ausführungsform der Erfindung gegenüber dem Stand der Technik zeigen.

### Kurze Beschreibung der Zeichnungen

Figur 1 zeigt eine Anlage zur Erzeugung von Dimethylether gemäß dem Stand der Technik in schematischer Darstellung
Figur 2 zeigt eine Anlage zur Erzeugung von Dimethylether gemäß einer Ausführungsform der Erfindung in schematischer Darstellung.

In den Figuren sind einander entsprechende Elemente mit identischen Bezugszeichen angegeben und werden der Übersichtlichkeit halber nicht wiederholt erläutert.

### Ausführliche Beschreibung der Zeichnungen

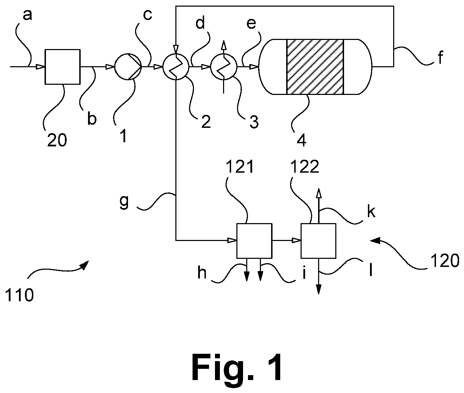

In Figur 1 ist eine Anlage zur Erzeugung von Dimethylether gemäß dem Stand der Technik schematisch dargestellt und insgesamt mit 110 bezeichnet.

Die Anlage 110 umfasst einen hier stark schematisiert dargestellten Synthesegasreaktor 20, der mit einem geeigneten Einsatz a, beispielsweise Erd- oder Biogas, beschickt werden kann. Dem Synthesegasreaktor 20 kann ein Synthesegasstrom b entnommen werden.

Der Synthesegasstrom b kann, gegebenenfalls nach einer Beimischung weiterer Ströme, mittels eines Verdichters 1 druckerhöht werden. Hierdurch kann ein für eine nachfolgende einschrittige Dimethylethersynthese erforderlicher Druck, beispielsweise ein Druck von 20 bis 100 bar, eingestellt werden.

Ein entsprechend verdichteter Strom, nun mit c bezeichnet, wird durch einen ersten Wärmetauscher 2 geführt, der mit einem Produktstrom f eines Reaktors 4 zur Synthese von Dimethylether (siehe unten) erwärmt werden kann. Der entsprechend erwärmte Strom d weist stromab des ersten Wärmetauschers 2 beispielsweise eine Temperatur von 200 bis 300°C auf. Der Strom d wird gegebenenf alls durch einen zweiten Wärmetauscher 3 geführt, welcher auch als Spitzenerhitzer bezeichnet wird.

Der in dem zweiten Wärmetauscher 3 weiter erwärmte Strom e wird in den Reaktor 4 eingespeist, der als Rohrreaktor ausgebildet ist und dessen Reaktionsrohre mit einem geeigneten Katalysator zur Einschrittsynthese von Dimethylether befüllt sind. Die Darstellung in der Figur 1 ist stark vereinfacht. Typischerweise sind Reaktoren 4 zur Synthese von Dimethylether stehend angeordnet, wobei ein Strom e bodenseitig in den Rohrreaktor 4 eingespeist wird. Kopfseitig wird dem Reaktor 4 ein Strom f entnommen.

Aufgrund der exothermen Reaktion in dem Rohrreaktor 4 liegt der Strom f bei nochmals höherer Temperatur vor. Der Strom f wird als Heizmedium durch den Wärmetauscher 2 geführt. Er kühlt sich hierdurch auf eine Temperatur ab, die beispielsweise ca. 30°C oberhalb der Temperatur de s verdichteten Stroms c liegt. Der entsprechend abgekühlte Strom, nun mit g bezeichnet, wird einer herkömmlichen Trennanlage 120 zugeführt. In der Trennanlage 120 werden aus dem Strom g beispielsweise unter zwischenzeitlicher Entspannung, Abkühlung, Rückverdichtung usw. (nicht dargestellt) in einem Schritt 121 ein Methanolstrom h und ein Wasserstrom i abgetrennt. Aus dem verbleibenden Rest werden in einem Schritt 122 die Ströme k und I gebildet, bei denen es sich beispielsweise um einen an Kohlendioxid angereicherten Strom k und einen an Dimethylether angereicherten Strom I handeln kann.

Die Zusammensetzung der Ströme k und I richtet sich nach der Zusammensetzung des Stroms g und den Betriebsparametern der Trennanlage 120. Wie bereits erläutert, lässt sich trotz der großen Siedepunktsunterschiede der beteiligten Komponenten aufgrund der guten Löslichkeit von Dimethylether und Kohlendioxid in Methanol/Wasser und dem vorliegenden hohen Druck mit einer einstufigen partiellen Kondensation keine befriedigende Trennung erreichen.

Soll in nachgeschalteten Trennschritten eine weitere Aufreinigung erfolgen, muss eine Abkühlung auf Temperaturen von deutlich unter 0°C erfolgen. Dies ist bei einem entsprechenden Wassergehalt des Stroms i jedoch nicht möglich, da das Wasser ausfrieren würde. Bei Anwesenheit von Methanol wäre keine befriedigende Trennung möglich. Eine Anwesenheit von Methanol ohne Wasser ("trockenem Methanol") ist zu vermeiden, da dieses die verwendeten Wärmetauscher schädigen würde. Entsprechendes gilt auch für eine ggf. erforderliche Abkühlung des Stroms I, der zunächst nur unbefriedigend getrennt vorliegt.

Vor diesem Hintergrund schlägt die vorliegende Erfindung, wie bereits erläutert, vor, einen Produktstrom, hier den Strom g, mit einer Temperatur oberhalb des Taupunkts in eine Absorptionskolonne einzuleiten und dort vorzutrennen.

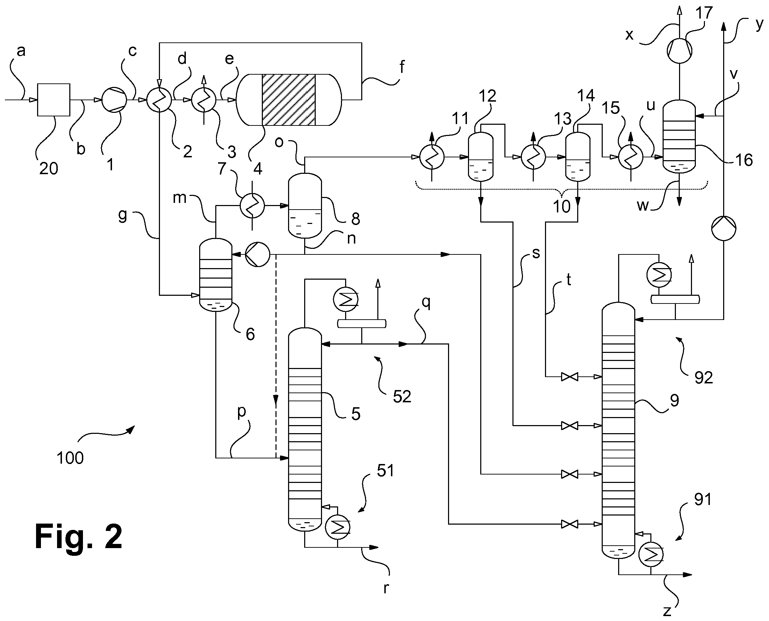

Dies ist in Figur 2 veranschaulicht, die eine Anlage zur Erzeugung von Dimethylether gemäß einer Ausführungsform der Erfindung zeigt. Diese ist insgesamt mit 100 bezeichnet.

Die Absorptionskolonne ist in der Figur 2 mit 6 bezeichnet. Wie bereits erläutert unterscheidet sich eine Absorptionskolonne 6 von einer Destillationssäule wie den nachfolgend erläuterten Destillationssäulen 5 und 9 unter anderem dadurch, dass sie keinen Sumpfverdampfer aufweist. In der Absorptionskolonne 6 aufsteigende Dämpfe werden durch einen am Kopf der Absorptionskolonne aufgegebenen Rücklauf gewaschen, so dass sich am Kopf der Absorptionskolonne die leichter flüchtigen und im Sumpf der Absorptionskolonne die schwerer flüchtigen Komponenten anreichern.

In der Anlage 100, die in Figur 2 dargestellt ist, wird der Strom g in die Absorptionskolonne 6 eingeleitet. Vom Kopf der Absorptionskolonne 6 wird ein Kopfstrom m entnommen und in einem Wärmetauscher 7 gegen ein geeignetes Kältemittel, beispielsweise Kühlwasser, gekühlt. Der entsprechend abgekühlte Strom m wird in einen Abscheiderbehälter 8 überführt, aus dessen Sumpf ein flüssiger Strom n entnommen und mittels einer Pumpe (ohne Bezeichnung) zumindest zum Teil als Rücklauf auf die Absorptionskolonne 6 aufgegeben wird.

Enthält der Strom g im dargestellten Beispiel neben Dimethylether auch Methanol, Wasser, Kohlendioxid, Kohlenmonoxid und Wasserstoff (neben Spuren anderer Verbindungen wie oben erläutert), gehen hiervon aufgrund der erläuterten Rückwaschung Dimethylether, Kohlendioxid, Kohlenmonoxid und Wasserstoff überwiegend in den Kopfstrom m über. Durch eine geeignete Abkühlung in dem Wärmetauscher 7 und entsprechende Abscheidebedingungen in dem Abscheiderbehälter 8 scheidet sich in dem Abscheiderbehälter 8 ein Sumpfprodukt ab, das im Wesentlichen aus Dimethylether und Kohlendioxid (gegebenenfalls mit Spuren von Methanol) besteht.

Vom Kopf des Abscheiderbehälters 8 kann ein Strom o gasförmig abgezogen werden, der, zusätzlich zu Kohlendioxid, Kohlenmonoxid und Wasserstoff, ebenfalls Dimethylether enthält. Der Strom o wird anschließend einer sequenziellen Abkühlung und Kondensation unterworfen, wie unten erläutert. Der nicht als flüssiger Rücklauf auf die Absorptionskolonne 6 aufgegebene Anteil des Stroms n kann bei geeigneter Zusammensetzung, wie die bei der sequenziellen Abkühlung und Kondensation des Stroms o anfallenden Kondensate, in eine Destillationssäule 9 ("weitere Destillationssäule") eingespeist werden. Anderenfalls erfolgt eine Einspeisung in eine Destillationssäule 5 ("erste Destillationssäule"), wie mit einem gestrichelten Flusspfeil veranschaulicht. Aus dem Sumpf der Absorptionskolonne 6 wird ein flüssiger Strom p entnommen und in die Destillationssäule 5 überführt.

Rücklaufmenge und Bodenzahl der Absorptionskolonne 6 können so optimiert werden, dass ein entsprechendes Sumpfprodukt p in nurmehr geringerer Menge anfällt. Vorteilhafterweise wird der Rücklauf, der auf die Absorptionskolonne 6 aufgebracht wird, derart eingestellt, dass der Methanol- und Wassergehalt in dem Strom m minimiert wird. Die sich auf diese Weise ergebende Zusammensetzung des Stroms o ist derart, dass sich in der Abkühlungs- und Kondensationssequenz 10, der der Strom o unterworfen wird, die zuvor erläuterten Nachteile, beispielsweise ein Ausfrieren von Wasser, nicht mehr einstellen können.

In der Destillationssäule 5, die mit einem Sumpfverdampfer 51 und einem Kopfkondensator 52 betrieben wird, kann der Strom p, der im Wesentlichen noch aus Methanol, Wasserstoff, Dimethylether und Kohlendioxid besteht, in einen im Wesentlichen aus Dimethylether und Kohlendioxid bestehenden Kopfstrom und einen im Wesentlichen aus Methanol und/oder Wasser bestehenden Sumpfstrom r getrennt werden. Ein Teil des Kopfstroms wird in dem Kopfkondensator 52 verflüssigt und auf die Destillationssäule 5 als Rücklauf aufgegeben. Ein weiterer verflüssigter Anteil des Kopfstroms wird im dargestellten Beispiel als Strom q abgezogen. Der nicht verflüssigte Rest wird im dargestellten Beispiel beispielsweise einer Verbrennung zugeführt. Der Strom q wird im Rahmen dieser Anmeldung als "Transferstrom" bezeichnet und in eine weitere Destillationssäule 9 überführt. Wie erwähnt, kann abweichend zur Darstellung in der Figur 2 ein dem Strom q entsprechender Transferstrom auch gasförmig bereitgestellt werden, insbesondere, wenn die erste Destillationssäule 5 auf einem höheren Druckniveau als die nachfolgend erläuterte weitere Destillationssäule 9 betrieben wird. Wird der Strom q in flüssiger Form bereitgestellt und liegt der Betriebsdruck der ersten Destillationssäule 5 unterhalb jener der weiteren Destillationssäule 9, kommt eine Pumpe zur Druckerhöhung zum Einsatz. Im umgekehrten Fall erfolgt eine Entspannung, beispielsweise über ein Ventil, wie in der Figur 2 dargestellt. In dem Kopfkondensator 5 nicht verflüssigtes Fluid, dessen Anteil bei einer Bereitstellung eines flüssigen Transferstroms q vorteilhafterweise minimiert wird, kann statt der thermischen Verwertung auch teilweise an geeigneter anderer Stelle in den Trennprozess zurückgeführt werden, wozu ggf. eine Verdichtung erfolgt. Die Art der Bildung des Transferstroms q ist nicht auf das hier veranschaulichte Beispiel beschränkt. Beispielsweise kann der Strom q auch direkt, d.h. unter Umgehung des Kopfkondensators 52, von der Destillationssäule 5 in die Destillationssäule 9 überführt werden. Auch der Sumpfstrom r kann an geeigneter Stelle verwendet werden. Abgeschiedenes Wasser kann auch zu einer Abwasserbehandlung oder einer Entgasung geführt werden.

Die bereits mehrfach erwähnten Schritte zur Weiterbehandlung des Stroms o sind hier insgesamt mit 10 angegeben. Der Strom o wird dabei zunächst einem Wärmetauscher 11 zugeführt und anschließend in einen Abscheiderbehälter 12 eingespeist. Die Abkühlung in dem Wärmetauscher 11 wird dabei derart vorgenommen, dass sich in dem Abscheiderbehälter 12 ein Kondensat s abscheidet. Ein in dem Abscheiderbehälter 12 gasförmig verbleibender Anteil wird einem Wärmetauscher 13 zugeführt und anschließend in einen weiteren Abscheiderbehälter 14 eingespeist. Auch dort wird ein Kondensat, hier mit t bezeichnet, erhalten.

Die Kondensate s und t werden, zusammen mit dem nicht auf die Absorptionskolonne 6 rückgeführten Anteil des Stroms n, in die bereits erwähnte weitere Destillationssäule 9 eingespeist, die wie unten erläutert betrieben wird. Ein auch am Kopf des Abscheiderbehälters 14 gasförmig verbleibender Anteil wird in einem weiteren Wärmetauscher 15 abgekühlt. Er liegt stromab dieses Wärmetauschers 15 beispielsweise bei einer Temperatur von -35°C oder darunter vor, beispielsweise knapp oberhalb der Schmelztemperatur von Kohlendioxid. Die Temperatur des Stroms o stromauf des Wärmetauschers 11 beträgt demgegenüber beispielsweise +35 °C. Der entsprechend abgekühlte Strom, hier mit u bezeichnet, wird im dargestellten Beispiel in eine weitere Absorptionskolonne 16 überführt. Dies ist optional.

Der Strom u enthält noch Dimethylether, Kohlendioxid, Kohlenmonoxid und Wasserstoff. Unter Verwendung eines flüssigen Rücklaufs v, der aus einem Teil eines Kondensats gebildet wird, das aus einem Kopfstrom der weiteren Destillationssäule 9 erhalten wird, wird im dargestellten Beispiel im Sumpf der Absorptionskolonne 16 ein Gemisch aus Dimethylether und Kohlendioxid abgeschieden. Am Kopf der Absorptionskolonne 16 wird hingegen ein Gemisch abgezogen, das im Wesentlichen aus Kohlendioxid, Kohlenmonoxid und Wasserstoff besteht. Dieses kann, gegebenenfalls nach entsprechender Verdichtung in einem Verdichter 17, als Strom x anderweitig verwendet werden. Die Verwendung der weiteren Absorptionskolonne 16 ist optional, es kann auch eine anderweitige Verarbeitung des Stroms u erfolgen.

In die weitere Destillationssäule 9 werden die Ströme s und t sowie der Transferstrom q eingespeist. Da diese unterschiedliche Gehalte an Dimethylether und Kohlendioxid aufweisen (Spuren von Kohlenmonoxid und Wasserstoff sind in gelöster Form ebenfalls enthalten), werden diese in unterschiedlicher Höhe in die Destillationssäule 9 eingespeist, wozu geeignete Ventile (ohne Bezeichnung) vorgesehen sind. In die weitere Destillationssäule q wird im dargestellten Beispiel auch der nicht in die Absorptionskolonne 6 rückgeführte Anteil des Stroms n eingespeist. Wie erwähnt, ist dies möglich, wenn dieser Strom wasser- und ggf. methanolfrei ist. Alternativ dazu ist auch eine Einspeisung in die erste Destillationssäule 5 möglich, wie mit einem gestrichelten Flusspfeil veranschaulicht.

Auch die weitere Destillationssäule 9 wird mit einem Sumpfverdampfer 91 und einem Kopfkondensator 92 betrieben. Ein Kopfstrom der weiteren Destillationssäule 9 wird in dem Kopfkondensator 92 unter Verwendung eines mit einem geeigneten Kältemittel betriebenen Wärmetauschers zumindest teilweise verflüssigt und als flüssiger Rücklauf auf die weitere Destillationssäule 9 aufgegeben. Ein weiterer Anteil wird zur Bildung des Stroms v und eines weiteren Stroms y verwendet.

Aus dem Sumpf der weiteren Destillationssäule 9 wird ein flüssiger Strom z entnommen, der im Wesentlichen noch aus Dimethylether besteht, jedoch insbesondere frei oder arm an Kohlendioxid ist.

## Patentansprüche

1. Verfahren zur trenntechnischen Bearbeitung eines zumindest Dimethylether, Methanol, Wasser, Kohlendioxid, Kohlenmonoxid und Wasserstoff enthaltenden Produktstroms (g) eines zur Synthese von Dimethylether aus Synthesegas (e) verwendeten Reaktors (4), wobei
- der Produktstrom (g) zumindest teilweise gasförmig in eine mit einem flüssigen Rücklauf betriebene Absorptionskolonne (6) eingespeist wird, wobei der Absorptionskolonne (6) kopfseitig ein gasförmiger Kopfstrom (m) und sumpfseitig ein flüssiger Sumpfstrom (p) entnommen wird,
- der Sumpfstrom (p) zumindest zum Teil in eine erste Destillationssäule (5) eingespeist wird, wobei der ersten Destillationssäule (5) ein Dimethylether enthaltender Transferstrom (q) und ein überwiegend Methanol und/oder Wasser enthaltender Strom (r) entnommen wird,
- der der Absorptionskolonne (6) entnommene Kopfstrom (m) zumindest zum Teil zunächst auf ein erstes und anschließend weiter auf ein oder mehrere weitere Temperaturniveaus abgekühlt wird, wobei nach dem Abkühlen auf das erste Temperaturniveau ein erster Kondensatstrom (n) gebildet wird und nach dem weiteren Abkühlen auf das oder die weiteren Temperaturniveaus ein oder mehrere weitere Kondensatströme (s, t) gebildet werden, und
- der erste Kondensatstrom (n) teilweise zur Bildung des flüssigen Rücklaufs verwendet wird und der oder die weiteren Kondensatströme (s, t) zumindest teilweise in eine weitere Destillationssäule (9) eingespeist werden, der sumpfseitig ein flüssiger Strom (z) entnommen wird, der überwiegend Dimethylether enthält und arm an oder frei von Kohlendioxid ist.

2. Verfahren nach Anspruch 1, bei dem ein nicht zur Bildung des flüssigen Rücklaufs verwendeter Anteil des ersten Kondensatstroms (n) zumindest teilweise in die erste Destillationssäule (5) oder die weitere Destillationssäule (9) eingespeist wird.

3. Verfahren nach einem der vorstehenden Ansprüche, bei dem der Produktstrom (g) auf einem Druckniveau von 20 bis 100 bar, insbesondere von 30 bis 70 bar, in die Absorptionskolonne (6) eingespeist wird.

4. Verfahren nach einem der vorstehenden Ansprüche, bei dem der Produktstrom (g) auf einem Temperaturniveau von 60 bis 150 °C in die Absorptionskolonne (6) eingespeist wird.

5. Verfahren nach einem der vorstehenden Ansprüche, bei dem das weitere Abkühlen des der Absorptionskolonne (6) entnommenen Kopfstroms (m) auf das oder die weiteren Temperaturniveaus ein Abkühlen bis auf ein minimales Temperaturniveau zwischen der Schmelztemperatur von Kohlendioxid und -15 °C umfasst.

6. Verfahren nach Anspruch 5, bei dem ein nach dem Abkühlen auf das minimale Temperaturniveau ein gasförmig verbliebener Anteil des der Absorptionskolonne (6) entnommenen Kopfstroms (m) in eine weitere Absorptionskolonne (16) eingespeist wird.

7. Verfahren nach Anspruch 6, bei dem die weitere Absorptionskolonne (16) mit einem weiteren flüssigen Rücklauf (v) betrieben wird, der aus einem verflüssigten, kohlendioxidreichen Kopfstrom der weiteren Destillationssäule (9) gebildet wird.

8. Verfahren nach einem der vorstehenden Ansprüche, bei dem der Produktstrom (g) 2 bis 50 Molprozent Dimethylether, 0,1 bis 20 Molprozent Methanol, 0,1 bis 20 Molprozent Wasser, 1 bis 50 Molprozent Kohlendioxid, 0,1 bis 25 Molprozent Kohlenmonoxid und 5 bis 90 Molprozent Wasserstoff enthält.

9. Anlage (100) zur Herstellung von Dimethylether (z) mit einem zur Synthese von Dimethylether (z) aus Synthesegas (e) eingerichteten Reaktor (4), mit einer Trennanlage, die zur trenntechnischen Bearbeitung eines zumindest Dimethylether, Methanol, Wasser, Kohlendioxid, Kohlenmonoxid und Wasserstoff enthaltenden Produktstroms (g) des Reaktors (4) eingerichtet ist, wobei die Trennanlage aufweist
- eine Absorptionskolonne (6), die dafür eingerichtet ist, mit dem Produktstrom (g) zumindest teilweise gasförmig gespeist und mit einem flüssigen Rücklauf betrieben zu werden, wobei Mittel vorgesehen sind, die dafür eingerichtet sind, der Absorptionskolonne (6) kopfseitig einen gasförmigen Kopfstrom (m) und sumpfseitig einen flüssigen Sumpfstrom (p) zu entnehmen,
- eine erste Destillationssäule (5) und Mittel, die dafür eingerichtet sind, den Sumpfstrom (p) zumindest zum Teil in die erste Destillationssäule (5) einzuspeisen und der ersten Destillationssäule (5) einen Dimethylether enthaltenden Transferstrom (q) und einen überwiegend Methanol und/oder Wasser enthaltenden Strom (r) zu entnehmen,
- Mittel, die dafür eingerichtet sind, den der Absorptionskolonne (6) entnommenen Kopfstrom (m) zumindest zum Teil zunächst auf ein erstes und anschließend weiter auf ein oder mehrere weitere Temperaturniveaus abzukühlen und nach dem Abkühlen auf das erste Temperaturniveau einen ersten Kondensatstrom (n) zu bilden und nach dem weiteren Abkühlen auf das oder die weiteren Temperaturniveaus einen oder mehrere weitere Kondensatströme (s, t) zu bilden, und
- eine weitere Destillationssäule (9) und Mittel, die dafür eingerichtet sind, den ersten Kondensatstrom (n) teilweise als den flüssigen Rücklauf zu verwenden, den oder die weiteren Kondensatströme (s, t) zumindest teilweise in die weitere Destillationssäule (9) einzuspeisen, und der weiteren Destillationssäule (9) sumpfseitig einen flüssigen Strom (z) zu entnehmen, der überwiegend Dimethylether enthält und arm an oder frei von Kohlendioxid ist.

## Claims

1. Process for separatory treatment of a product stream (g) containing at least dimethyl ether, methanol, water, carbon dioxide, carbon monoxide and hydrogen from a reactor (4) used for synthesis of dimethyl ether from synthesis gas (e), wherein
- the product stream (g) is fed at least partly in gaseous form into an absorption column (6) operated with a liquid reflux, wherein a gaseous tops stream (m) is withdrawn from the top, and a liquid bottoms stream (p) is withdrawn from the bottom, of absorption column (6),
- the bottoms stream (p) is at least partly fed into a first distillation column (5), wherein a dimethyl ether-containing transfer stream (q) and a predominantly methanol- and/or water-containing stream (r) are withdrawn from the first distillation column (5),
- the tops stream (m) withdrawn from the absorption column (6) is at least partly cooled, initially to a first temperature level and subsequently further to one or more further temperature levels, wherein after the cooling to the first temperature level a first condensate stream (n) is formed and after the further cooling to the further temperature level(s) one or more further condensate streams (s, t) are formed, and
- the first condensate stream (n) is partly used for forming the liquid reflux and the further condensate stream(s) (s, t) are at least partly fed into a further distillation column (9), from the bottom of which a liquid stream (z) is withdrawn which contains predominantly dimethyl ether and is poor in or free from carbon dioxide.

2. Process according to Claim 1, in which a fraction of the first condensate stream (n) not used for forming the liquid reflux is at least partly fed into the first distillation column (5) or the further distillation column (9).

3. Process according to either of the preceding claims, in which the product stream (g) is fed into the absorption column (6) at a pressure level of 20 to 100 bar, in particular of 30 to 70 bar.

4. Process according to any of the preceding claims, in which the product stream (g) is fed into the absorption column (6) at a temperature level of 60 to 150°C.

5. Process according to any of the preceding claims, in which the further cooling of the tops stream (m) withdrawn from the absorption column (6) to the further temperature level(s) comprises a cooling to a minimum temperature level between the melting point of carbon dioxide and -15°C.

6. Process according to Claim 5, in which a fraction of the tops stream (m) withdrawn from the absorption column (6) which remains gaseous after the cooling to the minimum temperature level is fed into a further absorption column (16).

7. Process according to Claim 6, in which the further absorption column (16) is operated with a further liquid reflux (v) formed from a liquefied carbon dioxide-rich tops stream from the further distillation column (9).

8. Process according to any of the preceding claims, in which the product stream (g) contains 2 to 50 mol percent of dimethyl ether, 0.1 to 20 mol percent of methanol, 0.1 to 20 mol percent of water, 1 to 50 mol percent of carbon dioxide, 0.1 to 25 mol percent of carbon monoxide and 5 to 90 mol percent of hydrogen.

9. Plant (100) for producing dimethyl ether (z) having a reactor (4) set up for synthesis of dimethyl ether (z) from synthesis gas (e), having a separating plant set up for separatory treatment of a product stream (g) from the reactor (4) containing at least dimethyl ether, methanol, water, carbon dioxide, carbon monoxide and hydrogen, wherein the separating plant comprises
- an absorption column (6) set up to be fed with the product stream (g) at least partly in gaseous form and to be operated with a liquid reflux, wherein means are provided which are set up for withdrawing a gaseous tops stream (m) from the top, and a liquid bottoms stream (p) from the bottom, of absorption column (6),
- a first distillation column (5) and means set up for at least partly feeding the bottoms stream (p) into the first distillation column (5) and withdrawing from the first distillation column (5) a dimethyl ether-containing transfer stream (q) and a predominantly methanol- and/or water-containing stream (r),
- means set up for at least partly cooling the tops stream (m) withdrawn from the absorption column (6) initially to a first temperature level and subsequently further to one or more further temperature levels and after the cooling to the first temperature level forming a first condensate stream (n) and after the further cooling to the further temperature level(s) forming one or more further condensate streams (s, t), and
- a further distillation column (9) and means set up for partly using the first condensate stream (n) as the liquid reflux, at least partly feeding the further condensate stream(s) (s, t) into the further distillation column (9) and withdrawing from the bottom of the further distillation column (9) a liquid stream (z) which contains predominantly dimethyl ether and is poor in or free from carbon dioxide.

## Revendications

1. Procédé de traitement de séparation d'un courant de produits (g) contenant au moins de l'éther diméthylique, du méthanol, de l'eau, du dioxyde de carbone, du monoxyde de carbone et de l'hydrogène d'un réacteur (4) utilisé pour la synthèse d'éther diméthylique à partir d'un gaz de synthèse (e), selon lequel
- le courant de produits (g) est introduit au moins en partie sous forme gazeuse dans une colonne d'absorption (6) exploitée avec un reflux liquide, un courant de tête gazeux (m) étant soutiré à la tête de la colonne d'absorption (6) et un courant de fond liquide (p) au fond,
- le courant de fond (p) est introduit au moins en partie dans une première colonne de distillation (5), un courant de transfert (q) contenant de l'éther diméthylique et un courant (r) contenant principalement du méthanol et/ou de l'eau étant soutirés de la première colonne de distillation (5),
- le courant de tête (m) soutiré de la colonne d'absorption (6) est refroidi au moins en partie tout d'abord à un premier niveau de température, puis davantage à un ou plusieurs niveaux de température supplémentaires, un premier courant de condensat (n) étant formé après le refroidissement au premier niveau de température et un ou plusieurs courants de condensat supplémentaires (s, t) étant formés après le refroidissement supplémentaire au ou aux niveaux de température supplémentaires, et
- le premier courant de condensat (n) est utilisé en partie pour la formation du reflux liquide et le ou les courants de condensat supplémentaires (s, t) sont introduits au moins en partie dans une colonne de distillation supplémentaire (9), au fond de laquelle un courant liquide (z) est soutiré, qui contient principalement de l'éther diméthylique et qui est pauvre en ou exempt de dioxyde de carbone.

2. Procédé selon la revendication 1, selon lequel une fraction non utilisée pour la formation du reflux liquide du premier courant de condensat (n) est introduite au moins en partie dans la première colonne de distillation (5) ou la colonne de distillation supplémentaire (9).

3. Procédé selon l'une quelconque des revendications précédentes, selon lequel le courant de produits (g) est introduit dans la colonne d'absorption (6) à un niveau de pression de 20 à 100 bar, notamment de 30 à 70 bar.

4. Procédé selon l'une quelconque des revendications précédentes, selon lequel le courant de produits (g) est introduit dans la colonne d'absorption (6) à un niveau de température de 60 à 150 °C.

5. Procédé selon l'une quelconque des revendications précédentes, selon lequel le refroidissement supplémentaire du courant de tête (m) soutiré de la colonne d'absorption (6) au ou aux niveaux de température supplémentaires comprend un refroidissement jusqu'à un niveau de température minimal compris entre la température de fusion du dioxyde de carbone et -15 °C.

6. Procédé selon la revendication 5, selon lequel une fraction du courant de tête (m) soutiré de la colonne d'absorption (6) restant sous forme gazeuse après le refroidissement au niveau de température minimal est introduite dans une colonne d'absorption supplémentaire (16).

7. Procédé selon la revendication 6, selon lequel la colonne d'absorption supplémentaire (16) est exploitée avec un reflux liquide supplémentaire (v), qui est formé à partir d'un courant de tête riche en dioxyde de carbone liquéfié de la colonne de distillation supplémentaire (9).

8. Procédé selon l'une quelconque des revendications précédentes, selon lequel le courant de produits (g) contient 2 à 50 pour cent en moles d'éther diméthylique, 0,1 à 20 pour cent en moles de méthanol, 0,1 à 20 pour cent en moles d'eau, 1 à 50 pour cent en moles de dioxyde de carbone, 0,1 à 25 pour cent en moles de monoxyde de carbone et 5 à 90 pour cent en moles d'hydrogène.

9. Unité (100) pour la fabrication d'éther diméthylique (z), comprenant un réacteur (4) conçu pour la synthèse d'éther diméthylique (z) à partir d'un gaz de synthèse (e), comprenant une unité de séparation, qui est conçue pour le traitement de séparation d'un courant de produits (g) contenant au moins de l'éther diméthylique, du méthanol, de l'eau, du dioxyde de carbone, du monoxyde de carbone et de l'hydrogène du réacteur (4), l'unité de séparation comprenant :
- - une colonne d'absorption (6), qui est conçue pour être alimentée avec le courant de produits (g) au moins en partie sous forme gazeuse et exploitée avec un reflux liquide, des moyens conçus pour soutirer un courant de tête gazeux (m) à la tête de la colonne d'absorption (6) et un courant de fond liquide (p) au fond étant prévus,
- une première colonne de distillation (5) et des moyens qui sont conçus pour introduire au moins en partie le courant de fond (p) dans la première colonne de distillation (5) et soutirer de la première colonne de distillation (5) un courant de transfert (q) contenant de l'éther diméthylique et un courant (r) contenant principalement du méthanol et/ou de l'eau,
- des moyens qui sont conçus pour refroidir le courant de tête (m) soutiré de la colonne d'absorption (6) au moins en partie tout d'abord à un premier niveau de température, puis davantage à un ou plusieurs niveaux de température supplémentaires, et pour former un premier courant de condensat (n) après le refroidissement au premier niveau de température et pour former un ou plusieurs courants de condensat supplémentaires (s, t) après le refroidissement supplémentaire au ou aux niveaux de température supplémentaires, et
- une colonne de distillation supplémentaire (9) et des moyens qui sont conçus pour utiliser le premier courant de condensat (n) en partie en tant que reflux liquide, pour introduire le ou les courants de condensat supplémentaires (s, t) au moins en partie dans la colonne de distillation supplémentaire (9), et pour soutirer au fond de la colonne de distillation supplémentaire (9) un courant liquide (z), qui contient principalement de l'éther diméthylique et qui est pauvre en ou exempt de dioxyde de carbone.
